# EUROPEAN PATENT SPECIFICATION

(11) **EP 2 229 148 B1**
(45) Date of publication and mention of the grant of the patent: **05.03.2014**
(21) Application number: 08859412.2
(22) Date of filing: 11.12.2008
(51) Int. Cl.: A61K 9/14

(54) **PROCESS FOR PREPARING A PARTICULATE AND CRYSTALLINE DRUG SUBSTANCE**
VERFAHREN ZUR HERSTELLUNG EINES PARTIKULÄREN UND KRISTALLINEN WIRKSTOFFES
PROCÉDÉ DE PRÉPARATION D'UN MÉDICAMENT PARTICULES ET CRISTALLINE

(30) Priority: 13.12.2007 EP 07123165
(43) Date of publication of application: 22.09.2010
(73) Proprietor: Novartis AG, 4056 Basel (CH)
(72) Inventor: MUHRER, Gerhard, CH-4002 Basel (CH); KIECKBUSCH, Thomas, 79539 Lörrach (DE); SINGH, Dilraj, CH-4058 Basel (CH); THAKUR, Ranjit, CH-4002 Basel (CH); SCHAFFLUETZEL, Kurt, CH-4058 Basel (CH); RASENACK, Norbert, 79576 Weil am Rhein (DE)
(74) Representative: McLean, Craig Sutherland
(86) International application number: PCT/EP2008/067364
(87) International publication number: WO 2009/074666

(56) References cited:
- EP-A- 1 348 431
- EP-A- 1 449 528
- EP-A- 1 834 624
- WO-A-00/25746
- WO-A-02/00199
- WO-A-2004/054545
- WO-A-2005/089718
- WO-A-2005/105043
- US-A1- 2004 037 785

## Description

This invention relates to the production of pharmaceuticals or, more specifically, a process for preparing a particulate and substantially crystalline glycopyrronium salt.

When preparing a drug substance for formulation and administration by inhalation, freshly prepared drug substance, often in the form of coarse grains, is commonly micronised i.e. mechanically pulverised to give particles whose mean particle size is suitable for inhalation. That mean particle size is typically less than 10 microns, preferably less than 5 microns, in mean diameter and the micronisation process is usually an air-jet-milling process.

Unfortunately micronisation generally and air-jet-milling in particular can cause deleterious changes in the crystalline drug substance including forming high energy surfaces, amorphous parts, dust and generating electrostatic charges. The problem is particularly acute for drug substances whose physicochemical properties make formulation difficult and thus require using the most thermodynamically stable crystalline form of that substance. Such crystalline drug substances tend to absorb water from the atmosphere and aggregate and/or agglomerate thus making further processing more difficult or at least less efficient.

There is therefore a need to provide an alternative process for preparing a particulate and substantially crystalline drug substance that avoids or at least diminishes the unwanted effects of conventional micronisation, particularly via dry milling processes. The process should preferably reduce the tendency of the drug substance to aggregate and/or agglomerate.

International patent application WO 2005/105043 discloses pharmaceutical compositions comprising the antimuscarinic agent glycopyrrolate.

European patent application EP 1834624 A1 discloses a medicincal composition and process for preparing the same.

International patent application WO 2004/054545 discloses a process for the preparation of sterile aqueous suspensions based on active ingredients in the form of micronised crystalline particles designed for administration by inhalation.

International patent application WO 2005/089718 discloses a process for micronisation of pharmaceutically active agents.

United States patent US 5858410 discloses pharmaceutical nanosuspensions for medicament administration as systems with increased saturation solubility and rate of solution.

Krause et al International Journal of Pharmaceutics 214 (2001) pages 21-24 discloses the production and characterisation of highly concentrated nanosuspensions by high pressure homogenisation.

Hecq et al International Journal of Pharmaceutics 299 (2005) pages 167-177 discloses the production and characterisation of nanocrystals for solubility and dissolution rate enhancement of nifedipine.

Surprisingly, it has been found that homogenising a substantially crystalline glycopyrronium salt suspended in an anti-solvent thereof at elevated pressure and drying the resulting drug particles gives a particulate drug substance in substantially pure and crystalline form. The suspension and the dried drug substance retain a substantially stable particle size over a long period of time and are thus suitable for further formulation. Surprisingly, no particle aggregation or agglomeration is observed which is in strong contrast to a dry micronised (jet-milled) powder of the same drug substance. It is particularly surprising that the homogenised suspension and particulate drug substance can be prepared without the presence of a stabilising agent.

Accordingly, the present invention relates to a process for preparing a particulate and crystalline glycopyrronium salt, the process comprising the steps of:
(a) suspending the crystalline glycopyrronium salt in an anti-solvent for that drug substance to give a suspension;
(b) homogenising the suspension at a pressure between 500 and 2000 bar to give drug particles that have a mean particle size of less than about 10 µm; and
(c) drying the drug particles to remove any residual anti-solvent.

Preferably the process is carried out in the absence of a stabilising agent yet still gives a suspension which after drying yields a substantially pure drug substance powder.

Preferably the suspension is homogenised at elevated pressure to give drug particles that have a mean particle size of less than about 7 µm, especially less than about 5 µm.

Preferably the elevated pressure is between 500 and 2000 bar, more preferably between 800 and 1500 bar, especially about 900 to 1300 bar.

Preferably the suspension is homogenised at a reflux temperature of between 1 to 30° C, more preferably between 3 and 20°C, especially between 9 and 15°C.

Preferably the suspension is homogenised at elevated pressure for 1 to 100 cycles, more preferably 20 to 75 cycles, especially about 50 cycles.

Preferably the glycopyrronium salt is glycopyrronium bromide, glycopyrrolate USP.

Preferably the suspension is homogenised at a pressure that gives micron-sized particles of glycopyrrolate that contain substantially no amorphous parts and no excipients.

Terms used in the specification have the following meanings:
"Amorphous" as used herein describes a disordered solid state, which may appear during manufacture of the drug substance (crystallization step, drying, milling) or the drug product (granulation, compression). The X-ray powder diffraction pattern of an amorphous solid exhibits no sharp peaks.
"Anti-adherent agent" as used herein means a material that reduces the cohesion between particles and prevents fine particles becoming attached to the inner surfaces of an inhaler device, or a mixture of such materials. Anti-adherent agents also include anti-friction agents or glidants, which give the powder formulation better flow properties in the inhaler. They usually lead to better dose reproducibility and higher fine particle fractions. Typical anti-adherent agents include amino acids such as leucine, phospholipids such as lecithin or fatty acid derivatives such as magnesium stearate or calcium stearate.
"Anti-solvent" as used herein means a solvent in which a particular drug substance is substantially insoluble. For example glycopyrrolate is substantially insoluble in acetone so acetone as an anti-solvent for glycopyrrolate.
"Glycopyrronium salt" as used herein is intended to encompass any salt form or counterion of glycopyrronium, including but not limited to glycopyrronium bromide (glycopyrrolate), glycopyrronium chloride, or glycopyrronium iodide, as well as any and all isolated stereoisomers and mixtures or stereoisomers thereof. Derivatives of glycopyrronium salts are also encompassed. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxy-benzoate, p-hydroxybenzoate, 1-hydroxynaphthalene-2-carboxylate, 3-hydroxynaphthalene-2-carboxylate, methanesulfonate and benzene-sulfonate.
"Mean particle size" is the average diameter of particles as measured by laser light diffraction. The x90 mean particle size is the mean particle size below which 90% of particles of a sample have a lower mean particle size. The x50 mean particle size is the mean particle size below which 50% of particles of a sample have a lower mean particle size. The x10 mean particle size is the mean particle size below which 10% of particles of a sample have a lower mean particle size.
"Stabilising agent" as used herein means a substance that stabilises a drug substance mainly in suspension. Typical stabilising agents are ionic or non-ionic surfactants (e.g. poloxamers) or polymers such as cellulose ethers, PVP or PVA.

Throughout this specification and in the claims that follow, unless the context requires otherwise, the word "comprise", or variations such as "comprises" or "comprising", will be understood to imply the inclusion of a stated integer or group of integers but not the exclusion of any other integer or group of integers.

The present invention relates to a process of preparing a particulate and substantially crystalline glycopyrronium salt that is suitable for administration by inhalation. The process involves suspending the substantially crystalline glycopyrronium salt in an anti-solvent thereof to give a suspension, homogenising the suspension at a pressure between 500 and 2000 bar to give drug particles that have a mean particle size of less than about 10 µm, and drying the drug particles to remove any residual anti-solvent. The resulting drug particles are surprisingly aggregation and agglomeration resistant, especially compared to jet-milled powders of the same substance.

High pressure homogenization (HPH) is a well established and widely used technology for the large scale production of emulsions, solid-lipid nanoparticles, and, most importantly, in food technology. The starting material is suspended in a carrier fluid as a first step, the suspension is pressurised typically between 500 and 2000 bar, and then the pressure is relaxed across static micro-channel interaction geometries such as Y- or Z-mixers, or across dynamic valves, thus inducing particle size reduction by cavitation, shear forces in the liquid phase, and particle-particle as well as particle-wall collisions. The homogenization step can be repeated as many times as necessary or until the desired average particle size and particle size distribution (PSD) is reached. HPH overcomes some of the most severe drawbacks of conventional dry and wet milling including contamination by abrasion. Dust formation and equipment clogging are also successfully avoided, particularly when dynamic particle size reduction micro-channel systems are used. In order to obtain a stable suspension, in described fields of use, the drug substance is usually co-suspended with certain excipients which function as a stabilizer agent prior to carrying out the high pressure homogenisation process.

In the first step of the process of the invention, a particulate and substantially crystalline glycopyrronium salt (optionally with a pre-reduced particle size) is suspended in an anti-solvent to give a suspension.

The process of the invention is especially useful for crystalline glycopyrronium salt whose physicochemical properties make conventional formulation difficult, especially in the production of dry powder formulations for administration by inhalation. In general such substances often have activated surfaces and sufficient chemical stability to withstand treatment at the temperature employed.

In a preferred embodiment of the process of the present invention the glycopyrronium salt is glycopyrronium bromide or glycopyrrolate.

Glycopyrrolate, which has the chemical name 3-[(cyclopentyl-hydroxyphenylacetyl)oxy]-1,1-dimethyl-pyrrolidinium bromide, is an antimuscarinic agent that is currently administered by injection to reduce secretions during anaesthesia and or taken orally to treat gastric ulcers. However it is more recently proving to be useful in treating respiratory diseases.

Glycopyrrolate has the following chemical structure:

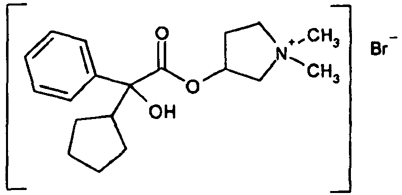

Glycopyrrolate is commercially available or can be prepared using the procedure described in United States patent US 2956062, the contents of which is incorporated herein by reference. It is preferably crystalline and contains minimal amorphous parts. Glycopyrrolate is particularly difficult to formulate as it tends to aggregate and agglomerate. This creates particular challenges when trying to formulate glycopyrrolate in dry powder formulations for administration by inhalation.

Glycopyrrolate has two stereogenic centres and hence exists in four isomeric forms, namely (3R,2'R)-, (3S,2'R)-, (3R,2'S)- and (3S,2'S)-3-[(cyclopentyl-hydroxyphenylacetyl)oxy]-1,1-dimethylpyrrolidinium bromide, as described in United States patent specifications US 6307060 and US 6,613,795. The contents of these patent specifications are incorporated herein by reference. The present invention embraces using one or more of these isomeric forms, especially the 3S,2'R isomer, the 3R,2'R isomer or the 2S,3'R isomer, thus including single enantiomers, mixtures of diastereomers, or racemates, especially (3S,2'R/3R,2'S)-3-[(cyclopentyl-hydroxy-phenylacetyl)oxy]-1,1-dimethylpyrrolidinium bromide.

The choice of anti-solvent is dictated by the choice of drug substance and related to the temperature at which the process is carried out. When the drug substance is glycopyrrolate, the anti-solvent is a solvent in which glycopyrrolate is substantially insoluble, for example acetone, propan-1-ol or ethanol, but preferably acetone. If the drug substance is micronised and formulated for use in a metered dose inhaler, homogenisation in hydrofluoroalkanes is feasible directly.

Solubility is determined by reference to the scale provided in The European Pharmacopoeia, i.e. if 10 to 30 parts solvent are required to solubilise one part of the drug substance the drug substance is said to be soluble in that solvent. Accordingly a solvent is anti-solvent of a given drug substance if more than 10 to 30 parts of that solvent are required to solubilise one part of the drug substance. Preferably the drug substance is sparingly soluble in the liquid medium employed. More preferably the drug substance is slightly, very slightly or even practically insoluble in the medium employed. Solubility is always dependent on the temperature at which the process is carried out and the temperature at which the final suspension is stored.

The drug substance is suspended in the anti-solvent using any suitable means, for example a mixing device, for example an ULTRA-TURAX homogeniser, a magnetic stirrer or other similar mixing device. Preferably the suspension does not contain a stabilising agent. This means that the pure drug substance is suspended in the liquid medium.

In the second step of the process of the invention, the suspension is homogenised at a pressure between 500 and 2000 bar to give drug particles that have a mean particle size of less than about 10 µm, preferably less than about 5 µm, but especially less than about 3 µm.

High pressure homogenisation is known to be an alternative milling technique. It produces a more homogeneous product than jet-milling, for example. However high pressure homogenization has hitherto been considered to provide such a high amount of energy to a drug substance that it would yield drug particles that are partially or even total amorphous (see Mueller et al, Nanosuspensionen- Formulierungen fuer schwerloesliche Arzneistoffe mit geringer Bioverfuegbarkeit: 1.Herstellung und Eigenschaften. Pharm. Ind. 1999,61, 74-78).

Conventional high pressure homogenisation processes are normally carried out in the presence of one or more stabilising agents and/or one or more excipients in order to prevent the newly created surface area against aggregation. The use of stabilising agents in particular in such processes appears to be highly preferred and at least usually essential. It is therefore surprising that in the process of the present invention the drug substance can be homogenised in the absence of any stabilising agents or excipients and yet remain stable over time without tending to aggregate and/or agglomerate or to show any change in particle size. This is very useful as the absence of any such stabilising agents or excipients facilitates downstream processing by avoiding the need to try to remove them later on. This also avoids contaminating the drug product with residual stabilising agents or excipients. While not wanting to be bound by theory, when there are no excipients present, newly created surfaces on the homogenised drug substances will not be covered by excipients and therefore one is not faced with the problem of excipient particles growing on the surfaces and detrimentally affecting the stability of the drug particles that are produced by the homogenisation.

The high stability of drug particles that are produced by the high pressure homogenisation allows the drug particles to be stored for much longer than drug particles produced by jet-milling.

The suspension is homogenised at elevated pressure to give drug particles that have a mean particle size of less than about 10 µm, preferably less than about 5 µm. In general, drug particles of that size are suitable for administration by inhalation. In certain embodiments the suspension is homogenised to give drug particles that have a mean particle size of less than about 7 µm. In other embodiments the suspension is homogenised to give drug particles that have a mean particle size of less than about 5 µm.

Particles having a mean particle size greater than about 10 µm are likely to impact the walls of the throat and generally do not reach the lung. Particles having a mean particle size in the range of about 2 µm to about 5 µm will generally be deposited in the respiratory bronchioles whereas smaller particles having a mean particle size in the range of about 0.05 µm to about 2 µm are likely to be exhaled or may be deposited in the alveoli and absorbed into the bloodstream.

Suitable pressures for homogenising the suspension will vary with the drug substance and the anti-solvent concerned. The elevated pressure at which the suspension is homogenised is between 500 and 2000 bar, preferably between 900 and 1500 bar, especially about 1000 bar. When the drug substance is glycopyrrolate and the anti-solvent is acetone, the elevated pressure at which the suspension is homogenised is preferably between 800 and 1500 bar, more preferably between 900 and 1200 bar, especially about 950 bar.

Suitable reflux temperatures for homogenising the suspension will vary with the drug substance and the anti-solvent concerned. In general, the reflux temperature at which the suspension is homogenised is preferably between 1 to 30° C, more preferably between 3 and 20°C, especially between 5 and 15°C. When the drug substance is glycopyrrolate and the anti-solvent is acetone, the reflux temperature at which the suspension is homogenised is preferably between 1 to 20°C, more preferably between 3 and 15°C, especially between 9 and 15°C.

Suitable number of cycles for homogenising the suspension will vary with the drug substance, the anti-solvent and the pressure concerned. In general, the suspension is homogenised for 1 to 100 cycles, more preferably 20 to 75 cycles, especially about 50 cycles. When the drug substance is glycopyrrolate and the anti-solvent is acetone, the suspension is homogenised for 1 to 100 cycles, more preferably 20 to 75 cycles, especially about 50 cycles.

Surprisingly, the process of the present invention yields a stable suspension and final /product without the use of any stabilising agents or excipients. However in certain embodiments it may be suitable for the drug substance to be homogenised together with a stabilising agent, particularly an anti-adherent agent, to further reduce the tendency for the drug substance to agglomerate and therefore improve the stability of the resulting drug substance. Preferably the anti-adherent agent is one or more metal stearates, one or more crystalline sugars or a mixture thereof. Especially preferred metal stearates include magnesium stearate and calcium stearate. Especially preferred crystalline sugars include lactose, more especially lactose monohydrate or anhydrous lactose. Other excipients can include surface active substances or polymers such as poloxamer, cellulose ethers, PVA, PVP and the like.

In the third step of the process of the invention, the drug particles are dried to remove any residual anti-solvent. This can be achieved using any art-known process such as freeze drying, spray drying or supercritical fluid drying. Preferably the drug particles are spray-dried. The resulting drug particles are surprisingly aggregation and agglomeration resistant and show a stable particle size over time.

If the anti-solvent is pharmaceutically acceptable the obtained suspension can also be used or further processed without any further drying operation.

Dry powder formulation made in accordance with the process of the present invention are stable and do not tend to aggregate and agglomerate over time.

In many cases where the drug substance contains amorphous parts prior to processing dry powder formulations of the present invention that are prepared from that drug substance show a noticeable, and often significant, reduction in amorphous parts.

Surprisingly, a suspension of glycopyrrolate, in particular, that is homogenised at elevated pressure in the absence of any excipients and then subsequently spray dried gives drug particles that are stable, do not contain any amorphous content and do not tend to aggregate or agglomerate.

Glycopyrronium salts that have been treated in accordance with the process of the present invention have a reduced tendency to agglomerate and thus provide a substantially stable solid bulk drug substance that facilitates further processing i.e. admixing of lactose carrier particles to give an inhalable dry powder. Such drug substances can be formulated for use in suitable devices including dry powder inhalers or metered dose inhalers. When the drug substance is to be delivered in a metered dose inhaler the drug substance is preferably resuspended in a suitable MDI-propellant. Alternatively the drug substance can be homogenised in a suitable MDI-propellant as suspending medium/anti-solvent.

The invention is illustrated by the following Examples.

### EXAMPLES

### Example 1

### Preparation of a dry powder of glycopyrrolate

### (a) Set-up of high pressure homogenisation equipment:

The equipment comprises a mixing vessel that is connected to a pneumatic piston pump. The outlet of the pump goes to a dynamic interaction valve which is connected to reflux line back to the mixing vessel. A heating/cooling jacket is provided in the mixing vessel that enables the temperature of the contents of the mixing vessel to heated or cooled. The homogeniser is a typical Microfluidics homogenizer that has been modified in the interaction chamber (from static IXC geometry to dynamic valve). In the process suspension from mixing vessel is pressurized to a defined pressure. This pressurized suspension is relaxed to atmospheric pressure across the dynamic valve, converting pressure energy to kinetic energy leading to cavitation, shear, particle to particle and particle to wall forces. These forces cause particle size reduction. The whole process is repeated until the desired average particle size is achieved.

### (b) Homogenisation of a suspension of glycopyrrolate in acetone:

16 g of coarse glycopyrrolate is suspended in 370 ml of acetone, and stirred for 60 minutes to achieve satisfactory dispersion. The suspension is then homogenised at a pressure drop of 950 bar, a reflux temperature of about 9-12.5° C, and a mixing/reflux vessel jacket temperature of 2° C. The piston pump stroke frequency is about 60, and the process is run for a total of 60 minutes, which is equivalent to a total of 48 passes or homogenisation cycles. Samples are also taken as in process control after 15 (12 passes) and 30 (24 passes) minutes, and analyzed along with the final product.

### (c) Spray-drying of glycopyrrolate particles:

The spray dryer is operated at an outlet temperature of around 75-78°C, i.e., above the boiling point of neat acetone, in order to avoid solvent condensation in the spray tower. The inlet temperature is set to 100°C, and the experiment initiated by spraying pure solvent. The spray rate is set to a flow of 5 ml/min using a Büchi lab scale spray dryer, with the aspirator operated at 100%. The nitrogen flow rate is kept constant at 600-700 l/h. At these operating conditions, the homogenised suspension is sprayed, and a dry powder is collected in a cyclone followed by
a cloth filter.

### (d) Analysis of glycopyrrolate materials:

Examination by scanning electron microscopy shows coarse glycopyrrolate are irregular crystals of about 50 to 100 µm. Product quality and particle size changes radically during the homogenisation step with morphology of glycopyrrolate changing from large, irregular size crystallites to more compact, platelet-like particles with an average size markedly below 5 µm. The crystallites appear to break into fairly regular, rectangularly shaped fragments of smaller size in the early stages of homogenization, which are subsequently comminuted and smoothed further. Particle size decreases further at an increasing number of homogenization cycles, after 24 passes the average particle size is around 1 µm already, and the particle edges are noticeably more rounded than initially or after only 12 passes. No significant changes are observed in particles isolated after 24 and 48 passes through the homogeniser.
Laser light diffraction particle size analysis reveals a x50 mean particle size (as hereinbefore defined) of 1.3 µm, a x10 mean particle size of 0.6 µm and a x90 mean particle size of 3.1 µm. Ultrasonication at 60W is applied for 480 seconds to break loose aggregates in the suspension prior to starting the particle size distribution measurement.

The spray dried glycopyrrolate is examined by scanning electron microscopy (SEM), and laser light diffraction (LLD) and does not reveal any significant change in particle size and shape upon spray drying compared to the material in suspension directly after high pressure homogenisation. Particles still appear as rectangularly shaped fragments with smoothed edges and an average size around or slightly above 1 µm. The specific surface area of the dried suspension (analysed by adsorption analysis, BET surface measurement) is determined to be 4.0 m2/g.

Laser light diffraction particle size analysis of the final product after drying reveals a mean particle size, x50, of 1.7 µm, at a narrow size distribution and an x90 value of 3.6 µm. Once again ultrasonication at 60W is applied for 480 seconds to break any loose aggregates in the product prior to starting the particle size distribution measurement.

The specific surface area of the final product by BET analysis is determined to be 4.0 m2/g. Analysing the final product using X-ray powder diffraction analysis (XPRD) and DSC shows no change in crystallinity compared to the coarse drug substance.

The final product after spray drying is re-characterized after 25 days of storage at ambient conditions as well as at 4°C. No substantial increase in the average size or broadening of the size distribution is observed, e.g., the particle size distribution after 25 days of storage at ambient conditions is x50=1.85 µm at x90=3.95 µm.

These data show that high pressure homogenised and spray dried glycopyrrolate avoids the severe aggregation that is observed in micronised and spray dried glycopyrrolate.

### Example 2

### Particle size distribution of a high pressure homogenized glycopyrrolate suspension

### (a) Set-up of high pressure homogenisation equipment:

This is the same as for Example 1.

### (b) Homogenisation of a suspension of glycopyrrolate in acetone:

-20 g of coarse glycopyrrolate is suspended in 320 ml of acetone, and stirred for 60 minutes to achieve satisfactory dispersion. The suspension is then homogenised at a pressure drop of 1150 bar, a reflux temperature of about 13-16° C, and a mixing/reflux vessel jacket temperature of 2° C. The piston pump stroke frequency is about 90, and the process is run for a total of 60 minutes, which is equivalent to a total of 80 passes or homogenisation cycles.

### (c) Spray-drying of glycopyrrolate particles:

The spray dryer is operated at an outlet temperature of around 75-78°C, i.e., above the boiling point of neat acetone, in order to avoid solvent condensation in the spray tower. The inlet temperature is set to 100°C, and the experiment initiated by spraying pure solvent. The spray rate is set to a flow of 5 ml/min using a Büchi lab scale spray dryer, with the aspirator operated at 100%. The nitrogen flow rate is kept constant at 600-700 1/h. At these operating conditions, the homogenised suspension is sprayed, and a dry powder is collected in a cyclone followed by a cloth filter.

The particle size distribution of samples of a suspension of glycopyrrolate prepared as described is measured by laser light diffraction using a HELOS particle size analyser with a 100 mm lense from Sympatec GmbH, Clausthal-Zellerfeld, Germany. The samples are measured after storage the suspension at either 4° C or room temperature for 1, 8, 10 and 12 weeks. Before taking any particle size distribution measurements the suspension is ultrasonicated at 60W for 480 seconds to break any loose agglomerates in the suspension. The results are shown in Table 1 below.

**TABLE 1**

| **Timepoint (storage period)** | **Storage temp.** | **x10 (µm)** | **x50 (µm)** | **X90 (µm)** |
|---|---|---|---|---|
| Directly after homog. | n.a. | 0.8 | 2.2 | 4.3 |
| 1 week | 4°C | 0.8 | 2.0 | 4.6 |
| 1 week | RT | 0.9 | 2.7 | 7.0 |
| 8 weeks | 4°C | 0.9 | 2.5 | 5.3 |
| 8 weeks | RT | 0.8 | 2.1 | 4.5 |
| 10 weeks | 4°C | 0.8 | 2.1 | 4.4 |
| 10 weeks | RT | 0.8 | 2.1 | 4.5 |
| 12 weeks | 4°C | 1.0 | 2.5 | 5.3 |
| 12 weeks | RT | 1.0 | 2.6 | 5.6 |

These data show the homogenised suspension of glycopyrrolate in acetone remains stable despite the absence of any stabilising excipients. They also show there is no significant change in particle size distribution of glycopyrrolate particles after spray drying.

### Example 3

### Stability of glycopyrrolate dry powder (obtained by spray-drying the suspension)

The stability of samples of spray dried glycopyrrolate prepared in accordance with Example 2 are measured by laser light diffraction using a HELOS particles size analyser with a 100 mm lens from Sympatec GmbH, Clausthal-Zellerfeld, Germany. Particles are analysed after 480 seconds of ultrasonication. The results are summarised in Table 2 below.

**TABLE 2**

| **Timepoint of storage*** | **x10 (µm)** | **x50 (µm)** | **x90 (µm)** |
|---|---|---|---|
| Directly after drying | 0.8 | 2.1 | 4.6 |
| 4 weeks | 0.8 | 2.0 | 3.9* |

| | | | |
|---|---|---|---|
| * storage conditions: 20°C / closed glass | | | |

These data show the dry powder samples of glycopyrrolate made in accordance with the process of the present invention remain substantially stable when stored in a closed glass at room conditions for four weeks in the absence of a stabilising agent.

### Example 4

### Comparison of a dry powder of glycopyrrolate prepared from a high pressure homogenised suspension of glycopyrrolate and a dry powder of glycopyrrolate prepared from micronised glycopyrrolate

1 kg of crystalline glycopyrrolate is co-micronised using a Hosokawa Alpine® 100 AFG fluid bed opposed jet mill with the following parameters: classifier speed, 17000 rpm; milling gas pressure, 4 bar. The mill is equipped with 3 nozzles of 1.9 mm diameter. The resulting micronised material shows a high tendency to aggregate and agglomerate which significantly hinders powder handling. As a consequence, the material has to be co-milled with a force control agent such as magnesium stearate in order to obtain a freely flowable non-agglomerating micronised drug substance.

In contrast, the high pressure homogenised and subsequently dried material (prepared as described in Examples 1, 2 and 3) shows no tendency to aggregate or agglomerate and can be further processed (for example into a dry powder inhalation formulation) more easily than jet-milled drug substance.

## Claims

1. A process for preparing a particulate and crystalline glycopyrronium salt, the process comprising the steps of:
(a) suspending the crystalline glycopyrronium salt in an anti-solvent for that drug substance to give a suspension;
(b) homogenising the suspension at a pressure between 500 and 2000 bar to give drug particles that have a mean particle size of less than about 10 µm; and
(c) drying the drug particles to remove any residual anti-solvent.

2. A process according to claim 1, wherein the pressure is between 900 and 1500 bar.

3. A process according to claim 1 or 2, wherein the suspension is homogenised at a reflux temperature of between 1 to 30°C.

4. A process according to any preceding claim, wherein the suspension is homogenised for 1 to 100 cycles.

5. A process according to any preceding claim, wherein the suspension is homogenised in the absence of a stabilising agent.

6. A process according to any preceding claim, wherein the drug particles are dried in the absence of a stabilising agent.

7. A process according to any preceding claim, wherein the crystalline glycopyrronium salt is glycopyrrolate.

8. A process according to claim 7, wherein the antisolvent is acetone, propan-1-ol or ethanol.

9. A process according to claim 8, wherein the antisolvent is acetone.

## Patentansprüche

1. Verfahren zur Herstellung eines partikelförmigen und kristallinen Glycopyrroniumsalzes, wobei das Verfahren die Schritte umfasst:
(a) Suspendieren des kristallinen Glycopyrroniumsalzes in einem Anti-Lösungsmittel für diese Arzneimittelsubstanz, um eine Suspension zu ergeben;
(b) Homogenisieren der Suspension bei einem Druck zwischen 500 und 2000 Bar, um Arzneimittelpartikel zu ergeben, die eine durchschnittliche Partikelgröße von weniger als ca. 10 µm aufweisen; und
(c) Trocknen der Arzneimittelpartikel, um jegliches restliches Anti-Lösungsmittel zu entfernen.

2. Verfahren nach Anspruch 1, wobei der Druck zwischen 900 und 1500 Bar beträgt.

3. Verfahren nach Anspruch 1 oder 2, wobei die Suspension bei einer Rückflusstemperatur von zwischen 1 bis 30 °C homogenisiert wird.

4. Verfahren nach irgendeinem vorhergehenden Anspruch, wobei die Suspension 1 bis 100 Zyklen lang homogenisiert wird.

5. Verfahren nach irgendeinem vorhergehenden Anspruch, wobei die Suspension in Abwesenheit eines Stabilisierungsmittels homogenisiert wird.

6. Verfahren nach irgendeinem vorhergehenden Anspruch, wobei die Arzneimittelpartikel in Abwesenheit eines Stabilisierungsmittels getrocknet werden.

7. Verfahren nach irgendeinem vorhergehenden Anspruch, wobei das kristalline Glycopyrroniumsalz Glycopyrrolat ist.

8. Verfahren nach Anspruch 7, wobei das Anti-Lösungsmittel Aceton, Propan-1-ol oder Ethanol ist.

9. Verfahren nach Anspruch 8, wobei das Anti-Lösungsmittel Aceton ist.

## Revendications

1. Procédé de préparation d'un sel de glycopyrronium particulaire et cristallin, le procédé comprenant les étapes de :
(a) mise en suspension du sel de glycopyrronium cristallin dans un anti-solvant pour cette substance médicamenteuse pour donner une suspension ;
(b) homogénéisation de la suspension à une pression entre 500 et 2 000 bars pour donner des particules de médicament qui ont un diamètre moyen de particule de moins d'environ 10 µm ; et
(c) séchage des particules de médicament pour éliminer tout anti-solvant résiduel.

2. Procédé selon la revendication 1, dans lequel la pression se situe entre 900 et 1 500 bars.

3. Procédé selon l'une des revendications 1 ou 2, dans lequel la suspension est homogénéisée à une température de reflux d'entre 1 à 30°C.

4. Procédé selon l'une quelconque des revendications précédentes, dans lequel la suspension est homogénéisée pendant 1 à 100 cycles.

5. Procédé selon l'une quelconque des revendications précédentes, dans lequel la suspension est homogénéisée en l'absence d'un agent stabilisant.

6. Procédé selon l'une quelconque des revendications précédentes, dans lequel les particules de médicament sont séchées en l'absence d'un agent stabilisant.

7. Procédé selon l'une quelconque des revendications précédentes, dans lequel le sel de glycopyrronium cristallin est le glycopyrrolate.

8. Procédé selon la revendication 7, dans lequel l'anti-solvant est l'acétone, le propan-1-ol ou l'éthanol.

9. Procédé selon la revendication 8, dans lequel l'anti-solvant est l'acétone.
